(19) **Europäisches Patentamt**

**European Patent Office**

**Office européen des brevets**

(11) **EP 0 522 824 B1**

(12) # EUROPEAN PATENT SPECIFICATION

(45) Date of publication and mention
of the grant of the patent:
**11.09.1996 Bulletin 1996/37**

(51) Int. Cl.$^6$: **A61F 13/04**, A61F 15/00

(21) Application number: **92306213.7**

(22) Date of filing: **07.07.1992**

(54) **Kit for applying orthopaedic bandages**

Ausrüstung zum Anbringen orthopädischer Verbände

Trousse pour l'application de bandages orthopédiques

(84) Designated Contracting States:
**AT BE CH DE ES GB IT LI LU NL**

(30) Priority: **08.07.1991 US 726477**

(43) Date of publication of application:
**13.01.1993 Bulletin 1993/02**

(73) Proprietor: **JOHNSON & JOHNSON ORTHOPAEDICS, INC.**
**Raynham, Massachusetts 02767-0350 (US)**

(72) Inventors:
• **Ferroni, John**
  **Delran, NJ 08075 (US)**
• **Holly, Henry**
  **No. Brunswick, NJ 08902 (US)**

• **Green, Richard**
  **Livingston, NJ 07039 (US)**
• **Oser, Zale**
  **Cranston, RI 02921 (US)**

(74) Representative: **Mercer, Christopher Paul**
**Carpmaels & Ransford**
**43, Bloomsbury Square**
**London WC1A 2RA (GB)**

(56) References cited:
WO-A-86/07533          DE-U- 8 524 580
FR-A- 0 741 988         FR-A- 2 644 341
GB-A- 0 971 552         US-A- 4 589 873
US-A- 4 774 937

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### Field of the Invention

The present invention relates to a kit for applying orthopaedic casting bandages of the type used to form orthopaedic casts. In particular the invention relates to a kit for applying orthopaedic bandages of the type having an uncured resin coating thereon.

### Background of the Invention

Plaster of paris casts have been used to immobilize body members for some time. These bandages are made by depositing plaster of paris on a reinforcing scrim material such as gauze. When the plaster of paris is dipped in water, reactions take place which result in the hardening of the cast material. Plaster of paris casts, however, suffer from a number of disadvantages. X-ray transmission through the cast to determine whether a fracture has properly set is extremely difficult. In addition, the cast is quite heavy and restricts the mobility of patients wearing the cast.

In order to overcome the disadvantages of plaster of paris casts, numerous attempts have been made to develop plastic or plastic reinforced materials as replacements for plaster of paris. U.S. patent numbers 3,241,501 and 3,881,473 disclose casts which are made with a flexible fabric impregnated with a polymer which is capable of being cured by ultraviolet light.

Other attempts to replace plaster of paris casts are disclosed in German Offenlegenschrift numbers 2353212 and 2357931, U.K. Patent #1,578,895, and PCT Application #WO 81/00671. These bandages are open weave fabrics coated with polyurethane prepolymers, that is, reaction products of isocyanates and polyols. The bandages are dipped into water in the same manner as the plaster of paris and then applied to the limb of the patient. The water causes the prepolymer to polymerize and form a rigid polymer structure.

More recently it has been found that in working with such materials having a prepolymer resin coating that the tackiness of the resin of the bandage can make working with the bandages difficult and cumbersome for the doctor. In an attempt to address this issue a glove lubricant comprised of water, sorbitol, mineral oil and silicon fluid has been sold by 3M Company, St. Paul, Minn., under the trade name CastCreme with instructions to apply the lubricant to the gloves of one applying an isocyanate-functional prepolymer coated cast after wrapping of the cast but before molding of the cast to avoid having the exposed casting material adhere to the gloves of the one applying the cast. This is disclosed in the background of U.S. Patent 4,667,661 and 4,774,937.

The '661 and '937 patents are directed to addressing the adherence issue by providing the resin itself with a lubricant. The curable resin coated sheet is prelubricated with a lubricant which is either a) bonded to the resin, b) added to the resin or applied to the surface of the coated sheet or c) provided in a combination of the bonding and surface application described. In many instances however, the tacky feature of the orthopaedic bandage is desirable. As by way of example when the applier is attempting to get the end of the bandage to stick to the surface of the bandage wrap in order to terminate the application of the bandage. The addition of lubricant in the resin permits relative slipping of the resin coated sheet and requires molding the cast in position and holding it in position to prevent slippage.

Coatings for substrates having a low coefficient of friction have been shown in U.S. patent 4,100,309 entitled "Coated Substrate Having a Low Coefficient of Friction Hydrophilic Coating and a Method of Making the Same". That reference describes a substrate which is coated with a polyvinyl pyrollidone-polyurethane inter polymer. In the method, a poly-isocyanate and a polyurethane in a solvent such as methyl ethyl ketone are applied to a substrate and the solvent evaporated. If the substrate is a polyurethane, only the polyisocyanate need be employed. Polyvinyl pyrollidone in a solvent is then applied to the treated substrate and the solvent evaporated. The substance and coated objects described in this reference are used in blood and body contacting environments. In order to lubricate the introduction of devices into openings in the body.

WO 86/07533 discloses an orthopaedic cast system comprising a fabric with a hardening quantity of an $\alpha$ cyanoacrylate monomer containing composition. A kit is also disclosed which comprises rubber gloves. However, the rubber gloves in use will stick to the fabric and make positioning of the fabric difficult.

US Patent 4589873 discloses a method for applying a hydrophilic coating to a polymeric substrate. It is stated that the coating may be applied to examination gloves but no reference is made to gloves for use in applying a cast.

### Summary of the Invention

The invention provides a kit for supplying an orthopaedic bandage material for use in immobilizing a patient's leg. The kit comprises a sheet having a curable resin coating thereon which sheet is laminable after activation and at least one glove having a hand portion, open cuff portion and finger portions which has a substantially dry coating thereon which coating is lubricous in use relative to said resin. By lubricous in use the present invention contemplates a coating which causes the glove to slip more easily over the resin coated sheet when applying the resin coated sheet than the glove would without the dry coating thereon.

In a preferred embodiment the kit provides a substrate such as an open weave fabric with a prepolymer coating thereon which is formed to be activated and polymerized after immersion in water. A further characteristic of the glove in the preferred embodiment is that it is more lubricous when wetted with water than it is in its dry state. By dry coating on the glove it is contemplated by the present invention that the coating itself is dry at some point prior to use. But during use the coating will be wetted by the immersion of the sheet into the activation bath.

The kit may be contained in a thermoformed package and preferably may contain two subpackages containing sheets having the curable resin coatings thereon and at least one glove but preferably a pair of gloves. In this manner at least two sheet portions are supplied to the user for immobilizing the patient's body or limb.

Therefore it is contemplated that the present invention includes a thermoformed package defining an inner volume, a subpackage contained in that thermoformed package which subpackage contains at least one sheet having a curable resin coating thereon which resin is activatable in order to provide a laminable property to the sheet after activation. The package would further include at least one pair of gloves each glove being made of an elastomeric material having a hand portion, open cuff portion and finger portions and which glove is provided with a substantially dry coating thereon which is lubricous relative to the resin when wetted. Again the resin is preferably activatable by immersion in water in order to complete polymerization of the majority of the material in the resin. The kit may include a subpackage which is made out of two sheets or walls which are heat sealed about their periphery in order to contain the resin coated sheet. These heat sealed walls are provided with a pair of openings on opposite sides of a weakened portion of the heat seal in order to provide ease of opening. It is easily seen that with the lubricous gloves already donned by the user of the equipment assistance in opening the package should be inherent in the package structure.

## Brief Description of the Drawings

The invention will now be described with reference to the accompanying drawings wherein:

Fig. 1 is a perspective view of a kit of the present invention;

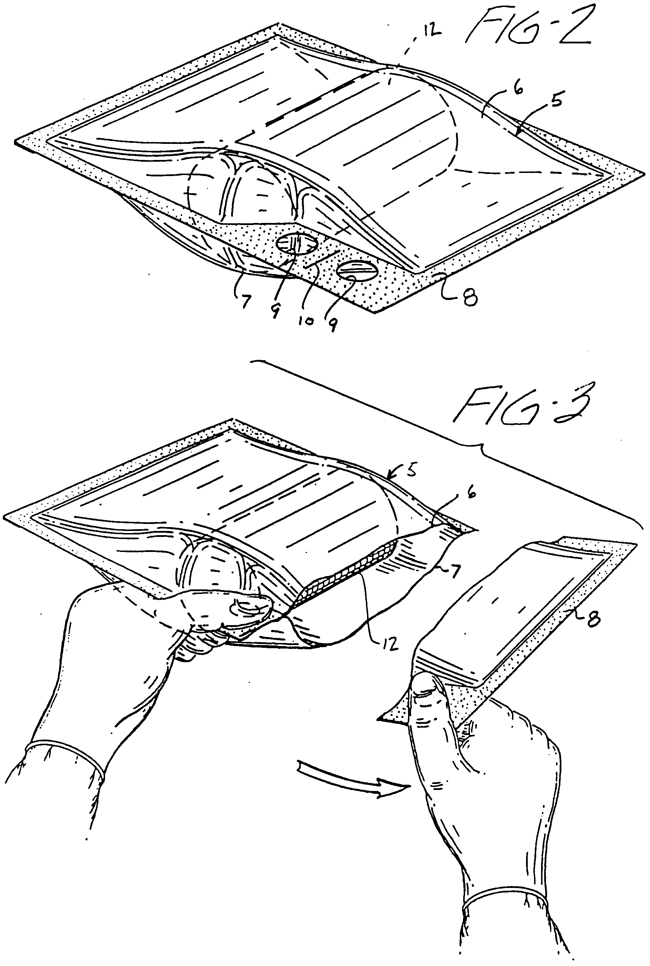

Fig. 2 shows a package containing a resin coated substrate for use as an orthopaedic bandage which is a part of the kit;

Fig. 3 shows the package of Fig. 2 being opened;

Fig. 4 depicts the glove of the kit of the present invention used in the method of the present application;

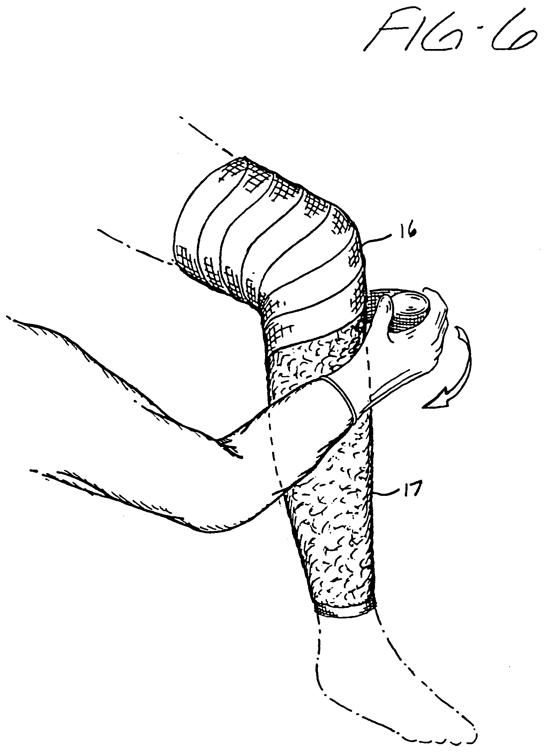

Fig. 5 shows the immersion of the orthopaedic material by the glove covered hand in a bath to activate the material;

Fig. 6 shows wrapping of the material about the limb of a patient in order to apply the material thereto.

## Detailed Description of the Invention

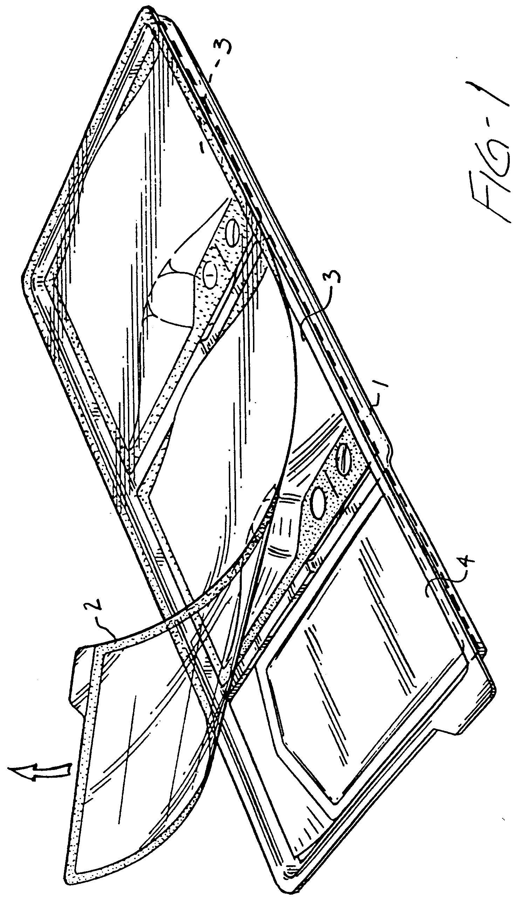

Figure 1 depicts a kit structure in which the supplies for the method of the present application are supplied. In particular a shell (1) having a peelable layer (2) defining a sealed inner volume is provided. The shell may be formed so as to provide as many compartments as necessary for providing the kit. In particular as shown in Figure 1 compartments (3) are provided for containing orthopaedic bandage material and compartment (4) is provided in order to supply at least one pair of coated gloves.

In use the package is opened by peeling peelable layer (2) away from shell (1) exposing the packages of orthopaedic bandage and gloves. The package (5) is removed and contains a roll of predetermined length of orthopaedic bandage material having uncured prepolymer resin deposited thereon. The bandage may be, for example, a knitted fiberglass substrate having a polyurethane prepolymer thereon such as the bandage sold by Johnson & Johnson Orthopaedics under the trade name Delta-Lite® fiberglass casting tape. The package (5) is formed of a top sheet (6) and bottom sheet (7) which are sealed about their periphery by, for example, heat sealing to form a unitary package. Sealed portion (8) widens at one corner to provide space to define openings (9) which are positioned on opposite sides of slit (10).

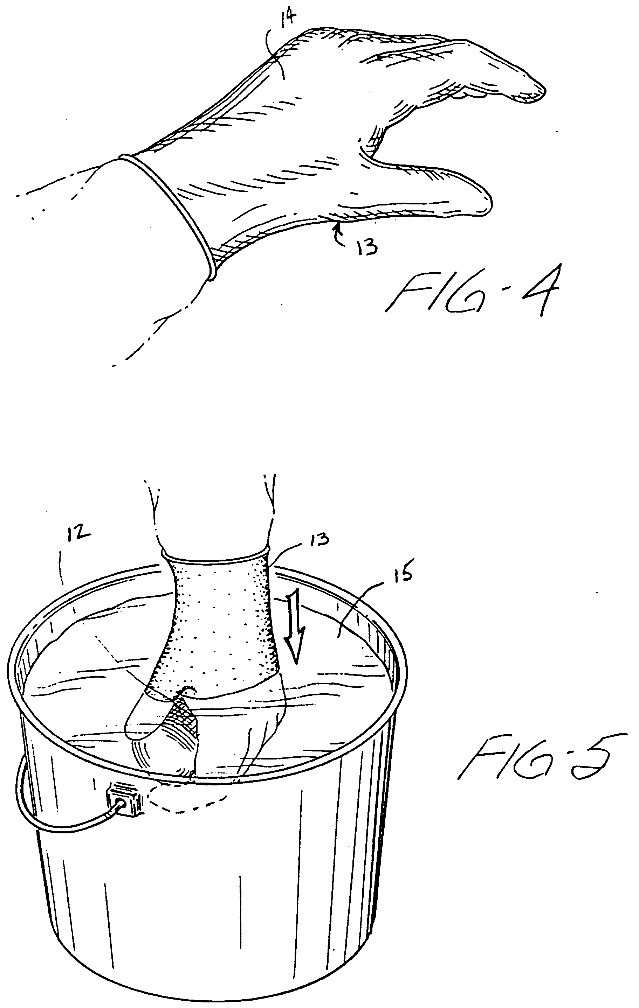

In use the package may be opened during the application of the orthopaedic bandage to the limb of the patient. In such a situation the applier would already be wearing the gloves of the invention, which will be described below, in order to provide a lubricous contact between the hands of the applier and the orthopaedic bandage material. In order to facilitate the opening of the package, openings (9) permit points at which the applier may grasp the package either by insertion of a finger or by contact of the fingers in a gripping fashion through the opening so that the hands are not slipping on the outer surface of the package. Slit (10) provides a start for the opening tear of the package as well as a stress point to facilitate opening of the package. A portion of the package is torn away as shown in Figure 3 and waste section (11) is discarded.

Thus opened the applier is provided access to a roll (12) of orthopaedic bandage material having thereon a prepolymer coating.

The kit contains a glove (13) having an outer surface (14) having thereon a substantially dry coating which forms a lubricous surface when wetted during the application of the bandage as is described below. This glove may be formed of any suitable substance such as for example butyl rubber, latex, polyvinyl chloride polyvinyl alcohol, neoprene or other natural or synthetic polymeric material.

The coating is applied to the glove substantially as described in U.S. patent 4,100,309. The coating may be, for example, polyvinyl pyrollidone which is applied in a non-water based solvent carrier as follows. Preformed latex rubber gloves are mounted in multiple clamping devices on a rack where the gloves are pressurized with air in order to partially inflate them and fully expose the gloves' outside surfaces. The rack is lowered into position so that the inflated gloves dip into a container of the solvent carrier solution for a period of time sufficient to completely wet the outside surfaces of the gloves. The rack, with attached gloves, is removed from its position above the dip bath and placed in an oven where solvent is allowed to evaporate and the resultant coating on the gloves is heated to facilitate curing.

The coating may also be provided in a water based fashion as follows. A machine for the continuous manufacture of latex rubber gloves is equipped with an additional dip tank positioned at the end of the line so that the latex gloves, prior to final drying, are subjected to an overdip of an aqueous based formulation in order to provide the coating to the glove, described above.

The coating applied to the gloves as an overdip at the end of the latex glove manufacturing line is applied from an aqueous bath whose solids (non-aqueous) components make up 14% of the total bath. These components are:

| Component | % of Total |
|---|---|
| 2-Pyrol (GAF) | 78.5 |
| Desmodur XP7005 (Mobay) | 1.8 |
| Glycerine (Dial) | 2.3 |
| Igepal CO-630 (Rhone Polenc) | 3.6 |
| Polyvinyl Pyrrollidone K-90 (GAF) | 11.5 |
| Kelco K7C233 (Kelco) | 1.4 |
| Impranil DLN (Mobay) | 0.4 |
| Neorez R-962 (ICI) | 0.5 |
| | 100.0 |

If a distinctively colored glove is desired, a pigment may be added to the above overdip bath or to the bath preceding the overdip bath on the production line. For example, if a light blue colored glove is desired one can add 0.02% Stan-Tone® 40WD01 Blue (Harwick Chemical Corp) to the latex substrate. This results in a finished glove with an attractive blue color, after the overdip coating has been accomplished.

Over Dip Coating Composition of Solids in Aqueous Systems

1. 2-Pyrol (GAF) - 2-pyrrolidone

2. Desmodur XP7005 (Mobay) - Blocked Aromatic Isocyanate Prepolymer
3. Glycerine (Dial Corp) - Glycerine
4. Igepal CO-630 (Rhone Polenc) - Surfactant Nonylphenyxy poly(ethyleneoxy) ethanol
5. PVP/K-90 (GAF) - Polyvinylpyrollidone
6. Kelco K7C233 (Kelco) - Sodium alginate (Hydrophilic colloid)
7. Impranil DLN (Mobay) - Anionic aqueous dispersion of an aliphatic polyester polyurethane
8. Neorez R-962 (ICI) - Aqueous dispersion of an aliphatic polyurethane

Upon donning glove (13) and opening the package as described above, access is gained to the orthopaedic bandage material. The orthopaedic bandage material is immersed in a bath (15) which may conveniently be water if a prepolymer is used which is activatable by immersing in water. The coating on the surface (14) of glove (13) is also in a manner activated by the water of the bath. The surface is such that when wetted by the bath it becomes significantly more lubricous than it was prior to being wetted. This lubricousness relative to the resin impregnated orthopaedic bandage permits the application of the bandage (16) over a stockinette (17) in a known manner. The bandage (16) adheres to itself in order to maintain its laminable position while at the same time being handled by a glove (13) having a lubricous surface which permits an easily slidable handling of the orthopaedic material by the glove covered hand.

Thus provided, the kit contains a resin coated bandage and a coated pair of gloves. The gloves have a coating which is lubricous relative the bandage when wetted. In this manner a kit having a glove with a coating matched to the resin may be provided.

**Claims**

1. A kit for providing an orthopaedic bandage material comprising:

    a) a sheet (16) having a curable resin coating thereon, which sheet is laminable after activation; and
    b) at least one glove (13) having a hand portion, open cuff portion and finger portions and having a substantially dry coating (14) thereon which is lubricious in use relative to said resin.

2. The kit of claim 1, which includes at least one pair of said gloves (13).

3. The kit of claim 1 or claim 2, which includes two such resin coated sheets (16).

4. The kit of any one of claims 1 to 3, wherein the sheet(s) (16) and glove(s) (13) are contained in a package (1).

5. The kit of claim 4, wherein the package (1) is a thermoformed.

6. The kit of claim 4 or claim 5, wherein the or each such resin coated sheet (16) is contained in a sub-package (5) within the package (1).

7. The kit of claim 6, wherein the or each sub-package (5) has an upper wall (6) and a lower wall (7), which walls are heat sealed about their peripheries to define an inner volume containing the sheet (16).

8. The kit of claim 7, wherein the heat sealing defines a pair of openings (9) on opposite sides of a weakened portion (10) of said heat sealing in order to provide a tear zone for the sub-package (5).

9. The kit of any one of claims 1 to 8, wherein the or each sheet (16) is activatable by immersion in water.

10. The kit of any one of claims 1 to 9, wherein the or each glove (13) becomes lubricious when wetted.

11. The kit of any one of claims 1 to 10, wherein said coating (14) on the or each glove (13) makes said glove (13) more lubricious relative to said resin than said glove (13) absent said coating (14).

12. The kit of any one of claims 1 to 11, wherein said coating (14) is hydrophilic.

13. The kit of any one of claims 1 to 12, wherein said coating (14) is polyvinylpyrrolidone.

14. The kit of any one of claims 1 to 13, wherein the or each glove (13) is made of an elastomeric material.

**Patentansprüche**

1. Ausrüstung zur Bereitstellung eines orthopädischen Bandagematerials bestehend aus:

   a) einer Binde (16), die eine härtbare Kunstharzbeschichtung besitzt, wobei die Binde nach der Aktivierung übereinandergeschichtet werden kann; und
   b) mindestens einem Handschuh (13) mit einem Handteil, einem offenen Ärmelteil und Fingerteilen sowie einer im wesentlichen trockenen Beschichtung (14) darauf, welche beim Einsatz gegenüber Kunstharz schlüpfrig wird.

2. Ausrüstung nach Anspruch 1, welche mindestens ein Paar Handschuhe (13) enthält.

3. Ausrüstung nach Anspruch 1 oder 2, welche zwei kunstharzharzbeschichtete Binden (16) enthält.

4. Ausrüstung nach Anspruch 1 bis 3, bei der die Binde(n) (16) und der (die) Handschuh(e) (13) in einem Päckchen (1) enthalten sind.

5. Ausrüstung nach Anspruch 4, bei der das Päckchen (1) thermisch geformt ist.

6. Ausrüstung nach Anspruch 4 oder 5, bei der die oder jede kunstharzharzbeschichtete Binde (16) in einem Innenpäckchen (5) innerhalb des Päckchens (1) enthalten ist.

7. Ausrüstung nach Anspruch 6, bei der das oder jedes Innenpäckchen (5) eine obere Lage (6) und eine untere Lage (7) besitzt, wobei die Lagen über ihren Umfang heißverklebt sind, um einen Innenraum zu bilden, der die Binde (16) enthält.

8. Ausrüstung nach Anspruch 7, bei der die Heißverklebung an den gegenüberliegenden Seiten eines abgeschwächten Teiles (10) der Heißverklebung ein Paar Durchbrüche (9) aufweist, um für das Innenpäckchen eine Reißzone vorzusehen.

9. Ausrüstung nach einem der Ansprüche 1 bis 8, bei der die oder jede Binden (16) durch Eintauchen in Wasser aktiviert wird.

10. Ausrüstung nach einem der Ansprüche 1 bis 9, bei der der oder jeder Handschuh (13) schlüpfrig wird, wenn er (sie) benetzt wird (werden).

11. Ausrüstung nach einem der Ansprüche 1 bis 10, bei der die Beschichtung (14) auf dem oder jedem Handschuh den(die) Handschuh(e) gegenüber dem Kunstharz schlüpfriger macht, als bei fehlender Beschichtung (14) auf dem(den) Handschuh(en) (13).

12. Ausrüstung nach einem der Ansprüche 1 bis 11, bei der die Beschichtung (14) wasseranziehend ist.

13. Ausrüstung nach einem der Ansprüche 1 bis 12, bei der die Beschichtung (14) aus Polyvinylpyrollidon besteht.

14. Ausrüstung nach einem der Ansprüche 1 bis 13, bei der der oder jeder Handschuh (13) aus einem elastomeren Material besteht.

**Revendications**

1. Trousse destinée à fournir une matière de bandage orthopédique comprenant :

   a) une feuille (16) revêtue d'une résine à même de faire prise, laquelle feuille pouvant être stratifiée après activation, et

b) au moins un gant (13) présentant une partie pour la main, une partie ouverte pour le poignet et une partie pour les doigts et présentant un revêtement (14) sensiblement sec qui est glissant lorsqu'il est utilisé par rapport à ladite résine.

2. Trousse suivant la revendication 1, comprenant au moins une paire dedits gants (13).

3. Trousse suivant la revendication 1 ou 2, comprenant deux feuilles revêtues de résine (16) de ce type.

4. Trousse suivant l'une quelconque des revendications 1 & 3, dans laquelle la ou les feuilles (16) et le ou les gants (13) sont contenus dans un emballage (1).

5. Trousse suivant la revendication 4, dans laquelle l'emballage (1) est un emballage thermoformé.

6. Trousse suivant la revendication 4 ou 5, dans laquelle la feuille (16) ou chaque feuille revêtue de résine (16) de ce type est contenue dans un sous-emballage (5) à l'intérieur de l'emballage (1).

7. Trousse suivant la revendication 6, dans laquelle le sous-emballage ou chaque sous-emballage (5) présente une paroi supérieure (6) et une paroi inférieure (7), lesdites parois étant thermosoudées sur leurs périphéries de manière & définir un volume intérieur contenant la feuille (16).

8. Trousse suivant la revendication 7, dans laquelle le thermosoudage définit deux ouvertures (9) de part et d'autre d'une partie de moindre résistance (10) dudit thermosoudage de manière à fournir une zone de déchirure pour le sous-emballage (5).

9. Trousse suivant l'une quelconque des revendications 1 à 8, dans laquelle la feuille ou chaque feuille (16) peut être activée par immersion dans l'eau.

10. Trousse suivant l'une quelconque des revendications 1 à 9, dans laquelle le gant ou chaque gant (13) devient glissant lorsqu'il est mouillé.

11. Trousse suivant l'une quelconque des revendications 1 à 10, dans laquelle ledit revêtement (14) sur le gant ou sur chaque gant (13) rend ledit gant (13) plus glissant par rapport à ladite résine que ledit gant (13) dépourvu du revêtement (14).

12. Trousse suivant l'une quelconque des revendications 1 à 11, dans laquelle ledit revêtement (14) est hydrophile.

13. Trousse suivant l'une quelconque des revendications 1 à 12, dans laquelle ledit revêtement (14) est du polyvinylpyrrolidone.

14. Trousse suivant l'une quelconque des revendications 1 à 13, dans laquelle le gant ou chaque gant (13) est constitué d'une matière élastomère.

FIG-1

FIG-2

FIG-3

FIG·4

FIG·5

FIG·6